# EUROPEAN PATENT APPLICATION

(11) **EP 4 671 351 A1**
(43) Date of publication of application: **31.12.2025**
(21) Application number: 24759816.2
(22) Date of filing: 21.02.2024
(51) Int. Cl.: C12G 1/022, C12M 1/04, C12M 1/107, B01D 50/00

(54) **METHOD AND SYSTEM FOR THE MACERATION OF WINES DURING FERMENTATION WITH RECOVERY OF SUBSTANCES RELEASED INTO THE ATMOSPHERE**

(30) Priority: 21.02.2023 ES 202330129
(71) Applicant: Aranda Gallego, José Manuel, 13600 Alcazar de San Juan (Ciudad Real) (ES); Mazo Gonzalez, José Ignacio, 02270 Villamalea (Albacete) (ES); Mazo Gonzalez, Javier Enrique, 02270 Villamalea (Albacete) (ES)
(72) Inventor: GARCÍA SEVERINO, Luis, 46370 CHIVA (VALENCIA) (ES); ARANDA GALLEGO, José Manuel, 13600 ALCÁZAR DE SAN JUAN (CIUDAD REAL) (ES); MAZO GONZÁLEZ, José Ignacio, 02270 VILLAMALEA (ALBACETE) (ES); MAZO GONZÁLEZ, Javier Enrique, 02270 VILLAMALEA (ALBACETE) (ES)
(74) Representative: Lehmann Novo, Maria Isabel
(86) International application number: PCT/ES2024/070102
(87) International publication number: WO 2024/175821

(57) **Abstract**

Method and system of maceration of wines in fermentation with recovery of substances released into the atmosphere, which includes a first stage of pre-fermentative cold maceration, which is carried out on crushed grapes and stored in a fermentation tank and where there is an injection of CO₂, in order to boost the dissolution of the aromas belonging to the fruity part of the grape; and a second stage where fermentation begins of the grape and the generation of CO₂ that carries aromas and alcohol; these substances are sucked out as they are generated; and, subsequently, they are injected back into the must contained in the fermentation tank and it is a system with which it manages to carry out a method of substance recovery in the fermentation of wines; and the stable and structured fixation of aromas in white wine and also colour in red wines.

## Description

### OBJECT OF THE INVENTION

The present invention relates to a new method of maceration of wines during fermentation by using CO₂ from fermentation and an associated system for the recovery of substances released into the atmosphere for the production of wines, which improves the quality of the product obtained and reduces the carbon footprint during the production process. To this end, the recovery method has the particularity of taking advantage of the solvent power of CO₂ and allowing the capture of the volatile substances released during the alcoholic fermentation of the must by means of an automated system and manages to obtain wines of high organoleptic quality through the stable fixation of aromas, in addition to the extraction and fixation of colourants in the case of red wines.

The field of application of the invention is within the wine sector, specifically it is oriented to a method and a system for the recovery of volatile substances released during the fermentation of wines.

### BACKGROUND TO THE INVENTION

It is known in the wine sector that, during the process of fermentation of the must, a large amount of CO₂ is produced as a residue of the transformation of sugar into alcohol by yeasts. In this fermentation process, along with this carbon and oxygen compound, numerous volatile compounds such as alcohols, aromas or similar are also dragged and released, which are very useful to obtain wines with great organoleptic quality.

Currently there are different systems that try to extract aromas and colour in wines, but they have serious drawbacks in terms of the stability of their fixation in the final product. The objective of most of these known systems during the fermentation of red wines is the abrupt breakage of the cap that is created inside the tanks, which causes the breakage of the skins that facilitates the extraction of colour. This procedure has the disadvantage that part of the pulp is deposited at the bottom of the tank, generating a large amount of lees.

On the other hand, other well-known systems have another disadvantage such as the use of gases from outside the fermentation process, for example, compressed air, oxygen and even CO₂, as vehicles to achieve the extraction of colour and aromas, which increases the energy and economic cost of fermentation, but without achieving the desired effects. In addition, in some of these systems, both the pressures at which they work and the amount of gas injected and the repetitions carried out, produce a large generation of lees, increasing workload in the winery and without achieving great variation or improvements in the organoleptic quality of the wines.

However, as described later in this descriptive report, the main advantage of the method and system that is the object of the invention over any known technology, is the recovery and stable fixation of aromas in the wine specific to each variety, together with a greater extraction of colour in red wines fixed in a stable way and all of this thanks to a innovative way of recovering, treating and injecting carbon dioxide at certain flows and pressures, without destroying skins; also avoiding the loss of alcohol and aromas released in fermentation, along with the capture of the CO₂ generated, which is the carrier for the other volatile substances.

Within the state of the technique, we highlight as processes and systems closest to the present invention those used to obtain red wines that have the sole objective of causing the cap to break once it has been formed inside the tank. The common objective sought by all these antecedents is the reduction of labour by producing the punch-down in an automated way and without the intervention of operators. Examples of these well-known systems are:
- the system called *"Oresteo"* (https://www.youtube.com/watch?v=zvcGoICC_3g), where the use of CO₂ absorbed from fermentation to carry out this process is disclosed, using high pressures; The use of these high pressures has the undesired consequence of the breakage of the skins;
- the system called *"Osiris"* (https://osirisforwine.com/), where the use of CO₂ for the breaking of the cap is disclosed, in a portable system that performs the punching down at high pressure; As in the previous case, having to resort to high pressures is common for the skins to break unwanted;
- the system called *"Ulises"* (https://www.agrovin.com/team/ulises/), where the use of air to produce the breakage of the cap at high pressure and performing macro-oxygenation is disseminated; This system has the disadvantage of generating a large amount of lees; and
- the system called "Air-mixing" (https://www.parsecsrl.net/en/air-mixing/), where the use of air to produce the breakage of the cap at high pressure and carrying out macro-oxygenation is disclosed.

There are also other systems for the recovery of aromas, such as the so-called "Re-Soul" (http://www.oenogreen.com/images/re-soul.pdf) whose purpose is only the recovery of aromas from fermentation, which are liquefied and stored in an external container and which may or may not later be incorporated into the finished wine. These aromas tend to oxidize, which can cause their degradation.

The method and system of recovery and fixation, in a stable and structured way of volatile substances in the fermentation of wines, object of this invention overcomes the inconveniences caused by the breakage of the cap inside the tank, the generation of lees, and the extraction of both aromas and colour in red wines, problems that may arise in the background previously indicated.

To solve these problems, the present invention has the particularity of using the main waste product of fermentation, which is the CO₂ of the fermentation process itself. In addition, with the methodology of the invention, the breakage of the skins is avoided, fixing the aromas and colour in a stable way, and practically without generating lees. These aspects make it possible to obtain high quality wines and represent considerable savings in both energy and costs in the winery together with the consequent reduction of the carbon footprint (especially if the surplus CO₂ is stored for reuse in the winery or for sale, applications beyond the scope of this invention). which is a technical advantage over known technologies.

Therefore, an advantage of the invention is the recovery and stable fixation of aromas until they are saturated in the wine, which would otherwise be lost. Another technical advantage achieved with the method and system of the invention in the case of red wines is greater colour extraction, taking advantage of the solvent power of CO₂, which is fixed in a stable way, avoiding its loss. In addition, as has already been pointed out in the object of the invention, this extraction occurs in such a way that the skins are not damaged, which means a reduction in work and energy costs in the wineries. Other added advantages are: avoiding the loss of alcohol and aromas released in fermentation and the capture of the CO₂ generated, which is the transport vehicle for other volatile substances.

The capture of the main waste product of the fermentation process, such as CO₂, is not only an advantage in itself since it avoids its release into the atmosphere reducing the carbon footprint, but it is also used according to the method and system of the invention to achieve a more efficient extraction and fixation of aromas in wines along with the colour in the case of red wines. In addition, the CO₂ left over from the invention's method is a reusable product for different tasks in the winery, such as the inerting of equipment, the protection of the grapes in the refrigeration with pellets at the entrance of the winery or similar, and the use for the generation of eco-fuels.

Another important advantage to highlight of the method and system object of this invention is the considerable energy savings in the fermentation process, especially in red wines, since it is not necessary to use pumps to carry out pumping over or "delestage", because with this methodology the compaction of the cap is avoided, causing the continuous mixing of the skins with the must using the CO₂ captured in the fermentation, which is injected according to pre-established parameters of density, together with other descriptive parameters of fermentation and depending on the volume of the tank, colour (white or red) at pressures and flows also pre-established according to the type of must.

For all these reasons, and in accordance with the above, the method and system of recovery of substances in the fermentation of wines of the invention resolves in a different and improved way what has been known so far in this sector, mainly in the capture of the volatile substances released during the alcoholic fermentation of the must to obtain wines of high organoleptic quality through the stable fixation of aromas in all types of wines; in addition to the stable fixation of colorants in red wines; and all this, reducing the carbon footprint in a totally different and improved way than what is known today.

A detailed description of the invention is made below, which completes these general ideas introduced in this point.

### DESCRIPTION OF THE INVENTION

The method of recovering substances in the fermentation of wines object of this invention, includes a first stage of cold pre-fermentative maceration, which is carried out on the crushed grape and stored in a tank at low temperature, avoiding the beginnings of fermentation, and then a second stage of fermentation takes place, where the fermentation of the grape and the formation of CO₂ is carried out along with other volatile substances that they carry mainly aromas and alcohol and where these substances are aspirated and condensed as they are generated to later be injected into the must in fermentation to it and other tanks that are in the pre-fermentative phase or fermentation phase in a uniform and homogeneous way.

The first cold pre-fermentative stage of the invention is based on achieving, in the absence of alcohol, a selective diffusion of water-soluble compounds of the grape, mainly the primary aromas, taking advantage of the solvent power of CO₂ to facilitate the extraction of colour, aromas, polysaccharides, polyphenols, etc.

At the start of the process in a first tank, as CO₂ is not yet available, it can be taken from any other tank that is fermenting in the winery or the pre-fermentation process (cold maceration) takes place in the traditional way. In other words, the crushed grapes introduced into this first tank are simply kept cold without any movement, since the cap has not formed, and pumping over is not possible. This initial pre-fermentation process can last approximately 24 hours, although it can vary depending on specific cases. After the pre-fermentation time that the winery's winemaker deems appropriate, yeasts and nutrients will be sown to achieve the start of fermentation. As soon as fermentation begins, the production of CO₂ will begin, which will be used for the method of the present invention.

Therefore, if you want to use CO₂ in pre-fermentation maceration from the very beginning, it is necessary to absorb CO₂ from another tank that has started fermenting. This CO₂ is then injected at a low flow rate into a second tank where crushed grapes have been placed prior to the start of fermentation.

Throughout this report, "low flow" (or "maintenance injection" is understood as the flow necessary to prevent compaction of the cap); and "high flow" (or "breakage" that is necessary to completely disintegrate the cap) to the percentages obtained according to the experiments or tests carried out in the winery, as indicated below (in both cases, at both low and high flow rates, the CO₂ injection pressure is in the order of 2-3 bar, which will be adjusted to the thickness of the cap determined by the height and diameter of each tank, a fact that allows us to overcome the disadvantages of other systems mentioned):
"High flow" is understood as the injection of CO₂ for the breakage of a skin cap inside a tank, when the injected volume of CO₂ with respect to the total volume of the tank is between 4% and 8% of CO₂. Preferably 4.7% of the volume of the tank for tanks up to 600 hl and 6% in tanks with a greater capacity of 600 hl.
"Low flow" is understood as the incorporation of CO₂ into a tank that prevents the caking of the skin contained inside, when the injected volume of CO₂ with respect to the total volume of the tank is between 0.4% and 1.2% of CO₂. This corresponds to approximately 10 - 15% of what is indicated in CO₂ injection for high-flow injection.

In the first pre-fermentation stage, the CO₂ injection is carried out at a low flow rate and is carried out for a pre-established time depending on the state of the grape. This time can be between 4 and 24 hours.

In a preferential realization, the method is applied to several tanks at the same time, so that when some tanks are fermenting, others are in the pre-fermentation stage, so there is always CO₂ available to inject, except for the first tank, in which CO₂ will not be injected until it is able to generate its own CO₂ once the fermentation process has started.

This pre-fermentation process is carried out at a low temperature, delaying the start of fermentation even without the addition of CO₂, with the lid of the tank open, thus preventing the cap from forming and at the same time expelling the vapours of sulphur compounds that are produced at the beginning of fermentation, which are harmful to the organoleptic characteristics of the wine.

The start of the second stage, fermentation, is determined by a decrease in sugar (density) that is detected by the analysis that is carried out automatically and that serves to initiate the opening of the carbonic suction valve. This start of fermentation is set by the winery's winemaker, acting mainly on the temperature (ceasing to introduce cold). As indicated above, after the pre-fermentation time that the winery's oenologist considers appropriate, yeasts and nutrients will be sown to achieve the start of fermentation. With fermentation, the production of CO₂ begins, along with other volatile substances that mainly carry aromas and alcohol, both sucked out and injected in a pre-established proportion according to the density, of the type of grape, causing the skins to be in continuous dynamic contact with the fermenting must in a uniform and homogeneous way in the tank throughout the fermentation process. In other words, of the CO₂ produced in a given tank in the fermentation phase, a part will be injected into another tank(s) in the pre-fermentation phase and another part will be injected into the same tank and/or other tanks that are in the fermentation phase.

In the pre-fermentation stage, the CO₂ injection will always be carried out at a low flow rate and in the fermentation stage, the injection can be at a low or high flow rate as necessary.

Throughout this description, "cap" is understood as the set of grape skins or peels that are clumped together in the upper part of a fermentation tank because they have a lower density than the liquid and are pushed upwards by CO₂.

Below is a table with the results of an exemplary test for a tank with a total volume of 600hl:

**Results of a 600-hl tank test**

| | |
|---|---|
| Capacity (litres) | 60000 |
| Total content (litres) | 51000 |
| Drained content (litres) | 38250 |
| Diameter (m) | 4 |
| Height (m) | 4,78 |
| Liquid cap volume (litres) | 11220 |
| Volume of dry cap (litres) | 8160 |
| Thickness of liquid cap (m) | 0,89 |
| Dry cap thickness (m) | 0,65 |
| Required pressure (bar) | 0,71 |

Once fermentation has started, the CO₂ to be used is absorbed from the tank itself along with the other volatile substances mentioned above, so that this mixture of CO₂ and volatile substances obtained from the fermentation tank is separated by means of a cyclone filter, obtaining on one hand CO₂ in a gaseous state and on the other hand volatile substances in a liquid state. The CO₂ is stored at a higher pressure (up to about 8 bar), and thus can be injected at different flows and pressures depending on the height of the tank and the thickness of the cap inside the fermentation tank itself (the maximum injection pressure being 3 bar). The condensed volatile substances are stored in a condensates tank, which are returned to the fermentation stage(s) at a low flow rate and at the pressure that is set according to the height of the tank.

The CO₂ suction of the fermenting tank is regulated by a motorized suction valve, prior to a turbine, which is activated by the PLC (Programmable Logic Controller), from now on PLC, based on the density measurement. Once the density is known, the sugar consumed in fermentation is calculated and the flow of CO₂ generated is obtained from an algorithm, so that the opening of the suction valve is adapted according to the CO₂ generated in each case, and taking into account the CO₂ that is being injected. It is important to automate this maneuver to avoid unnecessary oxygen aspiration during fermentation, since when injected together with CO₂ it would produce a faster proliferation of yeasts, higher fermentation speed implies an increase in temperature and more unwanted effects.

It should be noted that, in the case of red wines, during the fermentation process the skins tend to join or come together continuously, so that this cap does not compact, the continuous injection at a low flow rate of CO₂ is stopped from time to time and becomes an injection of CO₂ at a high flow rate that is between 4% and 8% of CO₂ with respect to the total of the volume of the fermentation tank, which causes the cap to break and the uniform distribution of the skins within the fermentation tank also causing a dynamic and continuous contact of the skins with the fermenting must. Once this injection of CO₂ at high flow is finished to achieve cap breakage, CO₂ is injected again at low flow rate continuously (which is between 0.4% and 1.2% of CO₂ injected with respect to the total volume of the fermentation tank), so that at this time of injection at low flow/pressure of CO₂, the volatile substances that are found in the fermentation tank are also injected in the system's condensates tank, as mentioned above.

Both low and high flow CO₂ injections are determined in both number and duration by the density measured. In this way, the skins are in continuous contact, without mistreating them, with the fermenting must, thus producing a greater extraction and fixation of aromas and colour. These depend on the type of grape and the wine-growing area, where density is taken into account, which is the main parameter that determines the injection times at low and high flow of CO₂, along with other descriptive parameters of fermentation.

According to usual practice, the time necessary for the proper development of the fermentation process of a wine is from 3 to 12 days. Therefore, the pre-established CO₂ injection time at low or high flow between different fermentation tanks in the fermentation phase is between 3 and 12 days.

On the other hand, as indicated above, high-flow CO₂ injections into the fermentation tank are mainly indicated for red wines where fermentation occurs with skins, but in the case of white wines where fermentation is usually carried out without skins, it does not need the cap to break as it does not exist. That is why in the case of white wines the CO₂ injected is only at a low flow rate, since the need for additional CO₂ injection is very small to keep the low-density substances moving and to add the condensed aromas, so it does not require a high-flow injection since there is no cap.

For all these reasons, in the fermentation stage of white wines, both CO₂ and volatile substances condensed are injected at low flow rates into the fermentation tank, which have previously been absorbed from the tank itself.

Below is a table where both low and high flow CO₂ injections for cap breakage are noted when they are performed based on the measured values of density expressed in kg/m3.

**Injection at "low flow" depending on density**

| | |
|---|---|
| 1130 > density > 1050 | Continuous injection |
| 1050 > density > 1030 | Continuous injection |
| 1030 > density > 1010 | Injection 10 minutes and 5 minutes stop |
| 1010 > density > 995 | Injection 5 minutes and 5 minutes stop |

**"High flow" injection for cap breakage depending on density**

| Note: *When the high-flow CO₂ injection occurs, the low-flow injection is stopped to devote all the compressor's work to this operation.* | |
|---|---|
| 1130 > density > 1050 | 4 operations |
| 1050 > density > 1030 | 3 operations |
| 1030 > density > 1010 | 2 Operations |
| 1010 > density > 995 | 1 Operation |

These operations are programmed in the PLC (Programmable Logic Controller) and can vary according to the characteristics that the winemaker wants to imprint on his wines, more freshness, more structure, the figures that are presented here, are the appropriate ones in general. However, the winemaker could vary these parameters as a greater expert of his wines, production and land where they are grown.

Another important parameter in the method of recovering substances in the fermentation of wine of the invention is the fermentation temperature that prevents the aromas of the wine from being lost since at a lower temperature the fermentation is slower and there is less evaporation to preserve aromas. Now, as mentioned above, the lowering of temperature is intended to avoid the loss of substances by evaporation, but with this invention it is possible to increase efficiency, since we keep the temperature low and what is carried by the CO₂ returns back to the tank. This parameter depends on the wine-growing area and whether the wine is red or white; and in the case of red wines, the fermentation temperature is higher than in white wines, to take advantage of the solvent factor of the temperature. As follows:
- The maximum fermentation temperature that red wines should not exceed is 28 ºC; preferably 26ºC.
- The maximum fermentation temperature that white wines should not exceed is 20 ºC; preferably 18ºC.

On the other hand, the measurement of redox potential (ORP) is another important parameter to take into account in the method of the invention that relates the density of the grape with the fermentation temperature and the kinetics of fermentation. This parameter indicates the possible problems regarding the reduction of the product, allowing it to be kept within the fermentation tank within the appropriate limits that prevent the degradation of sulphur to hydrogen sulphide and also prevents aroma's oxidation. The measurement of the ORP makes it possible to control the need for oxygen supply to increase the speed of fermentation, thus avoiding the reduction of aromas.

Below is a table of tests where the density of the grapes is related to the action on the fermentation temperature and the possible oxygen supply, with the density expressed in kg/m³ and the drop in temperature expressed in ºC. This temperature is generally lowered when fermentation is too fast and rises if it is slow to boost the yeasts.

**Acting on the fermentation temperature as a function of density**

| | |
|---|---|
| Density > 1075 and drop ≤ 0.025 | Does not act |
| Density > 1075 and drop ≥ 0.025 | Lower temperature, do not provide oxygen |
| Density > 1050 and ≤ 0.020 drop | Does not act |
| Density > 1050 and drop ≥ 0.025 | Lower temperature, do not provide oxygen |
| Density > 1030 and drop ≤ 0.020 | Does not act |
| Density > 1030 and drop ≥ 0.025 | Lower temperature, do not provide oxygen |
| Density > 1075 and drop ≤ 0.005 | Raising the temperature, providing oxygen |
| Density > 1075 and drop ≥ 0.005 | Does not act |
| Density > 1050 and drop ≤ 0.004 | Raising the temperature, providing oxygen |
| Density > 1050 and ≥ 0.004 drop | Does not act |
| Density > 1030 and drop ≤ 0.002 | Raising the temperature, providing oxygen |
| Density > 1030 and drop ≥ 0.002 | Does not act |

Thus, a high drop in density in a given time, corroborated by a low ORP, determines that action must be taken to reduce the temperature. Whereas, if the drop in density is small and the ORP rises, the recommendation would be to raise the temperature and even add oxygen to facilitate the growth of the yeasts. Therefore, based on the measured values of density and ORP, evaluating their trend in a 24-hour interval and taking into account the relationship between both parameters, the temperature must be acted on in the corresponding way and even oxygen must be added so that the fermentation speed is adequate.

Below is a table of tests where the redox potential (ORP, which is the verification of what the density indicates) is related to the action on the fermentation temperature and the possible oxygen supply, where the numerical values related to the density of the grape are expressed in kg/m³; numerical values related to the redox potential are expressed in mV; and all numerical values related to fermentation temperature are expressed in ºC.

**Action on fermentation temperature according to the ORP**

| **For the purposes of ORP** | |
|---|---|
| Density > 1075 and ORP > -30 | Does not act |
| Density > 1075 and ORP < -30 | Lower temperature, do not provide oxygen |
| Density > 1050 and ORP > -30 | Does not act |
| Density > 1050 and ORP < -30 | Lower temperature, do not provide oxygen |
| Density > 1030 and ORP > -30 | Does not act |
| Density > 1030 and ORP < -30 | Lower temperature, do not provide oxygen |
| Density > 1075 and ORP < +120 | Does not act |
| Density > 1075 and ORP > +120 | Raising the temperature, providing nutrients |
| Density > 1050 and ORP < +120 | Does not act |
| Density > 1050 and ORP > +120 | Raising the temperature, providing nutrients |
| Density > 1030 and ORP < +120 | Does not act |
| Density > 1030 and ORP > +120 | Raising the temperature, providing nutrients |

In this sense:
- the minimum temperature should not fall below 20 ºC in red wines and 14 ºC in white wines;
- the maximum temperature should not rise above 28 ºC, preferably 26 ºC in red wines and 20 ºC, preferably 18 ºC in white wines;
- you have to see the trend in the last 6 hours, and if the trend is constant, you should lower it by two degrees until the product goes down;
- no more than 1 degree should be lowered every 2 tests; y
- The trend followed between three analyses should give rise to action.
- The analyses are carried out every 2 hours, automatically
- The analyses are evaluated over a 24-hour time interval

Once the method that is the object of this invention has been explained, a system for the recovery of substances in the fermentation of wines, also the object of the invention, is described below. This system manages to develop a method of recovering substances in the fermentation of wines, such as the one previously described, for its implementation in wineries and with which it is possible to solve the existing technical problems in the state of the method.

The system of the invention includes one or more fermentation tanks, which is where the fermentation of the must is carried out, which are in connection with suction means (turbines or compressors) whose flow is regulated by the values obtained from the density measurement and are responsible for sucking the CO₂ generated in the fermentation plus that which is added in the process along with the rest of the volatile substances generated in fermentation. These suction media is connected to a cyclone filter, where a large part of the CO₂ substances are separated. The cyclone filter can be preceded by a heat exchanger to cool the fluid and thus improve separation performance. The CO₂ gas passes into a low-pressure buffer tank (approximately 0.4 bar) and the volatile substances that have been transformed into a liquid are transferred to the condensates tank, remaining available when a suitable level is reached to be returned to the fermentation tank(s) to which it is connected. This would be the part corresponding to the collection of volatile substances that are dragged along with the CO₂. In the first way of realization, the buffer tank containing CO₂ at low pressure (0.4 bar) is connected to the supply of at least one compressor where the CO₂ is compressed, up to a pressure of approximately 8 bar for storage (to have sufficient flow at the pressure necessary to cause the cap to break without breaking the skins). The compressor in turn is connected to a high-pressure CO₂ tank where the CO₂ is stored, so that this carbon dioxide is injected back into the fermentation tanks at a pressure of approximately 2 - 3 bar, since the high-pressure CO₂ storage tank is connected to the aforementioned tanks by means of injection. In a second embodiment mode, the CO₂ is also injected from the high-pressure tank into the fermentation tanks via injection media, although in this second embodiment mode the buffer reservoir is not present. In other words, in the second mode of realization, the compressor performs the dual function of sucking and compressing CO₂. This is the part corresponding to facilitating the extraction of the coloring matter together with the aromas that are in contact with the skins. For all these reasons, both volatile substances and CO₂ are injected into one or more fermentation tanks in a uniform and homogeneous manner.

As mentioned above, it is usual for a winery to have more than one fermentation tank, so the CO₂ that is generated in one or more of the tanks that are in the fermentation phase can be recovered, stored and reinjected into one or more tanks that are in the pre-fermentation or fermentation phase.

Another detail to highlight about the system of the invention is that the entire system as a whole is automated through a PLC (Programmable Logic Controller), which receives the data obtained by the different sensors located in the measuring station, where each sample extracted from the fermentation tanks is automatically analyzed. In other words, the fermentation tanks are connected to an automatic sampler system in connection with a measuring station where the density, temperature and redox potential of each tank are analyzed, so that this data is sent to the Programmable Logic Controller, which defines the state of the wine fermentation and manages these parameters according to their preset programming, such as the values indicated in the previous test tables.

The fermentation tank(s) are connected to suction media, where CO₂ and other volatile substances generated in the fermentation tank(s) are sucked out. In the first way of realization, the suction means consist of a suction turbine which is a side-channel vacuum pump with the capacity to suction the CO₂ produced by fermentation plus the added CO₂. In a second mode of realization, the suction media consist of a compressor that will perform in this case the double function of sucking the CO₂ from the fermentation tanks and compressing it at high pressure to be stored and then reinjected back into the fermentation tanks after passing through the cyclone filter (since in this mode of realization a high flow of CO₂ is not required since the fermentation tanks are of small volume).

In connection with the suction media there is a cyclone filter, where there is a first separation of the volatile substances from CO₂ in the order of 50%-60% of the total volatile substances aspirated and transported, and it is where the substances of the CO₂ are separated on one hand prior to the passage to the low-pressure buffer tank, and on the other hand, a second part of the volatile substances are condensed in the low-pressure tank through an existing coil in it prior to the passage to the condensate tank, in this stage between 20-30% of the condensates lost in must fermentation are recovered.

A heat exchanger can be advantageously installed between the suction media and the cyclone filter to cool the absorbed CO₂ together with the other substances to increase the efficiency of the cyclone filter. In this case, outcomes set out in the previous paragraph will be increased, and values close to 80% of separation may be reached.

In a first mode of realization, the compressor that is in connection with the low-pressure buffer tank compresses the CO₂ that receives, and sends it to the CO₂ tank until it reaches the pressure to which it is limited. In a possible realization, this compressor is an oil-free, food-grade compressor.

So, in the first mode of realization, both the CO₂ tank and the condensates tank are connected by means of an injection system to at least one fermentation tank, so that:
- the injection of CO₂ at a low flow rate into the fermentation tank occurs continuously, except when the control Programmable Logic Controller indicates that the cap is broken and then the injection enters at a high flow rate; and
- The injection of condensed aromas and alcohol occurs at a low flow rate when the quantity stored in the condensate tank reaches a pre-established level to be injected by means of a dosing pump for this purpose into the fermentation tank or tanks.

In a second mode of implementation, the operation will be the same, with the only difference that the low-pressure buffer tank is dispensed with and the suction media (in this case a compressor) directly compress the CO₂ (after passing through the cyclone filter) to the high-pressure CO₂ tank that is connected to at least one fermentation tank through an injection system.

Moreover, the PLC to which the data of each fermentation tank is received through the sensors of the measuring station, and which in turn is in connection with the CO₂ tank, manages the injection of CO₂ both at low and high flow through the density parameters together with the temperature and redox potential parameters received from the measuring station. Thus, low-flow injection occurs continuously except: when the density is low and stops are required or when it is necessary to perform a high-flow injection to avoid compaction of the cap, in the case of red wines.

To complete the description that is being made and in order to help a better understanding of the characteristics of the invention, a set of drawings is accompanied as an integral part of it where the following has been represented for illustrative and non-limiting purposes:
Figure 1 is a schematic representation of the system of recovery of substances in the fermentation of wines of the invention according to a first mode of realization.
Figure 2 is a schematic representation of the system of recovery of substances in the fermentation of wines of the invention according to a second mode of realization.

### DESCRIPTION OF A WAY OF REALIZING THE INVENTION

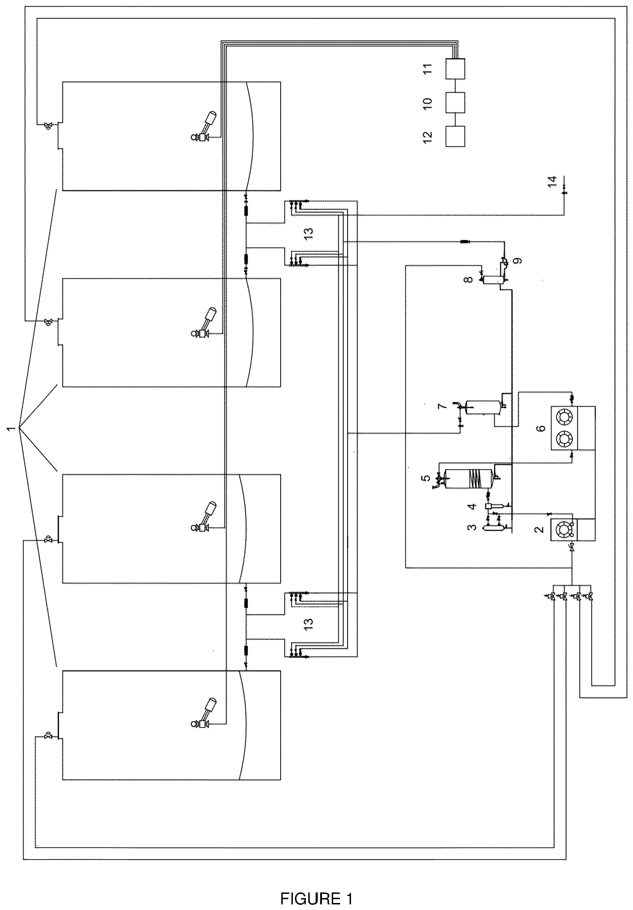

As can be seen in the Figure 1, in the first way of carrying out the system of recovery of substances in the fermentation of wines of the invention, the system comprises:
One or more fermentation tanks (1), which is where the fermentation of the must takes place and which are connected to a suction media (2) consisting of an aspiration turbine where the CO₂ is sucked together with the rest of the volatile substances generated in the fermentation.

The suction media (2) consisting of a suction turbine are in turn connected to a cyclone filter (4), where the CO₂ and volatile substances are separated. The cyclone filter can be preceded by a heat exchanger (3) to improve separation performance. The separated CO₂ is conducted and stored in a low-pressure buffer tank (5), while the volatile substances are conveyed to a condensate tank (8) where they are stored.

The low-pressure buffer tank (5) is connected to a compressor (6) where the CO₂ is compressed, which is also connected to a CO₂ tank (7) where the compressed CO₂ is stored at higher pressure. From this CO₂ reservoir (7) the carbon dioxide is injected back into the fermentation tanks (1) as the CO₂ reservoir (7) is also connected to the aforementioned reservoirs.

The carbon dioxide is injected back into the fermentation tanks (1) through injection system (13) comprising a set of automated valves placed at the bottom of the tanks that are automatically operated for the time necessary at each moment of fermentation for both high and low flow rates. as well as at least one pressure regulator that adapts it according to the tank to which it must be introduced. In an example of preferential realization, the set of automated valves would be four: one for high-flow, one for low-flow, one more for aromas and the last one for O₂ injection if necessary. All of them are sized according to the size and number of the existing tanks to inject the necessary flow during the time that is programmed. In the accompanying exemplifying figures, four pipes are shown: 2 lines for CO₂ at high and low flow rates (which could be one if instead of a solenoid valve for each case a variable pitch line is placed for both, since they would never act at the same time), another for aromas, and another for the injection of O₂ (14) in case necessary.

On the other hand, the volatile substances stored in the condensates tank (8) once a predetermined level has been reached are always injected at a low-flow rate by at least one dosing pump (9) back into the fermentation tank(s) (1), which are connected to the condensates tank (8) through that dosing pump (9) and the injection media (13);

In addition, the system of the invention also includes a measuring station (10) that integrates density, temperature and redox potential sensors that are connected to one or more fermentation tanks (1) through an automatic sampler (11). The samples taken from the fermentation tank(s) (1) arrive at this measuring station (10) where they are analyzed; and in connection with the same measuring station (10) there is a PLC (12) that is programmable, where the measured data of density, temperature and redox potential of or from the fermentation tanks (1) are received automatically and processed. Obviously, if it was desired to simplify the system and reduce costs, the density, temperature and ORP parameters could be entered manually by screen or other means equivalent to the PLC by an operator.

The Programmable Logic Controller (12) that is connected to the CO₂ tank (7) and to the injection media (13) shall regulate the injection of CO₂ at both high and low pressure and flow into the fermentation tank(s) (1) according to pre-established injection values and times as programmed, all as previously indicated in this descriptive report. The PLC is also connected to the dosing pump (9) for the return of aromas to the fermentation tanks (1) according to the program that determines how much is injected into each tank. Likewise, the PLC is connected to the system for external supply of O₂ (14) if necessary, regulating this addition in an automated way.

Figure 1 is a generic schematic representation of a substance recovery system in the fermentation of wines of the invention valid for white, rosé and red wines. These systems must logically be sized and adapted not only to the type of wine, but also to the size and number of tanks.

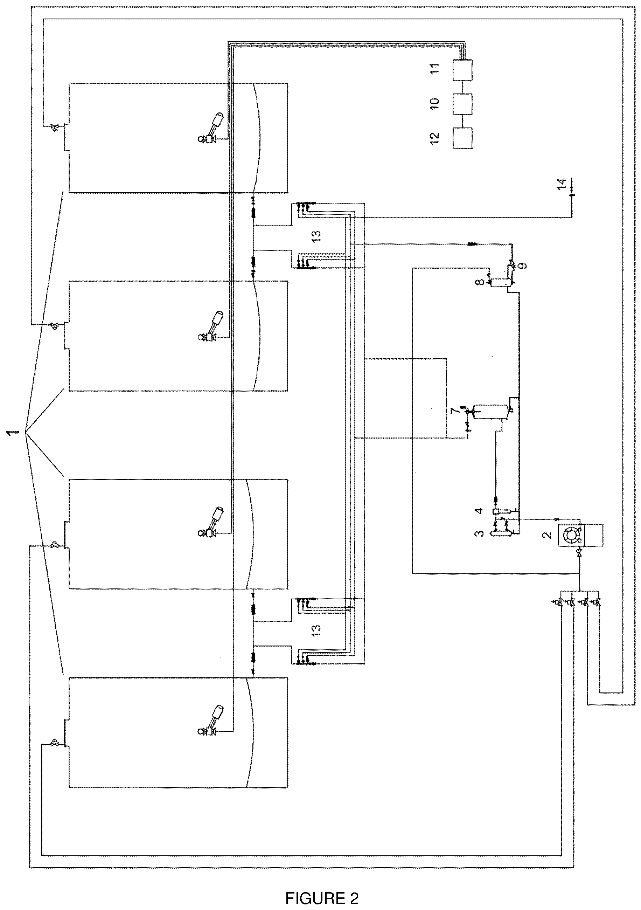

The figure 2 shows a second method of implementation, which would correspond to a possible system of recovery of substances in the fermentation of wines according to the method of the invention valid for small productions of wine (small tanks, one or two tanks, etc.) where the production of CO₂ in fermentation is much lower and therefore the CO₂ flows to be moved are much lower. This second method of implementation is a simplification of the first method of implementation, where the suction turbine is not installed, since in this type of small installation, the means of suction (2) will consist of a compressor that can perform the functions of suction and compression of CO₂. Preferably, this compressor will be of the oil- and silicone-free type and for food use.

Likewise, the low-pressure buffer tank (5) has been eliminated and the compressed CO₂ is stored in the high-pressure CO₂ tank (7) from where it is re-injected into the fermentation tanks (1) at both low and high flow managed by the PLC (12) that acts on the injection media (13) so that it is carried out at the appropriate pressure (controlled by pressure regulators) and for the necessary time. In other words, as in the first mode of realization, in this second variant the injection of CO₂ and aromas is controlled in a similar way by the PLC, which activates the solenoid valves for the injection of CO₂ after passing through pressure regulators for high and low flow, and also activates the aroma dosing pump once a predetermined level is reached and whenever we are injecting CO₂ at low flow.

## Claims

1. Method of maceration of wines in fermentation with recovery of substances released into the atmosphere, which is **characterized by** the fact that comprises:
- a first stage of pre-fermentative cold maceration, which is carried out on crushed grapes and stored in a fermentation tank with an injection of CO₂ at a low flow rate that is between 0.4% and 1.2% of CO₂ injected with respect to the total volume of the fermentation tank; and, where, in the absence of alcohol, a diffusion of water-soluble compounds from the grape takes place, and the solvent properties of CO₂ extracts aromas, polyphenols, polysaccharides or similar from the grape, and
- a second stage of fermentation, where the fermentation of the grape juice and the formation of CO₂ takes place together with other volatile substances that carry aromas and alcohols; where CO₂ and these substances are inhaled as they are generated; and, where, subsequently, these substances are injected again into the fermenting must contained in the same fermentation tank, or in other tanks that are in the pre-fermentation or fermentation phase, in a uniform and homogeneous manner, for a pre-established period of time.

2. Method of maceration of wines in fermentation with recovery of substances released into the atmosphere, according to claim 1, which is **characterized by** the fact that:
- the pre-established CO₂ injection time at low flow between different fermentation tanks in the pre-fermentation phase is between 4 and 24 hours, and
- the pre-established time for the injection of CO₂ at low or high flow between different fermentation tanks in the fermentation phase is between 3 and 12 days, depending on the duration of fermentation.

3. Method of maceration of wines in fermentation with recovery of substances released into the atmosphere, according to claim 1 or 2, where in the second stage of fermentation of red wines to prevent the skins from compacting and forming a cap:
- in a first step, the continuous injection of CO₂ at a low flow rate into the fermentation tank is stopped, and it becomes an injection of CO₂ at a high flow rate that is between 4% and 8% of CO₂ injected with respect to the total volume of the fermentation tank; and
- in a second step, CO₂ is injected again at a low flow rate, which is between 0.4% and 1.2% of CO₂ injected with respect to the total volume of the fermentation tank, continuously and together with the injection of the volatile substances, into the fermentation tank.

4. Method of maceration of wines in fermentation with recovery of substances released into the atmosphere, according to any of the above claims that is **characterized by** the fact that both at low and high flow the CO₂ injection pressure ranges between 2 and 3 bar depending on the thickness of the cap determined by the height and diameter of each tank.

5. Method of maceration of wines in fermentation with recovery of substances released into the atmosphere, according to claim 1 which is **characterized by** the fact that the aspiration of the mixture of CO₂ and volatile substances from the fermenting tank is regulated by a motorized suction valve, prior to a turbine, operated by a PLC or controller from the measurement of the density.

6. Method of maceration of wines in fermentation with recovery of substances released into the atmosphere, according to claims 1 or 5 which is **characterized by** the mixture of CO₂ and volatile substances aspirated from the fermentation tank being passed through an cooling system for refrigeration prior to separation by means of a cyclone filter, on one hand CO₂ and on the other hand volatile substances; where the CO₂ is stored under pressure in a buffer tank so that it can be injected at different flow rates and pressures depending on the height of the tank and the thickness of the cap inside the fermentation tank(s); and where the volatile substances are condensed and stored in a condensates tank, which are returned to the fermentation stage or tanks.

7. Method of maceration of wines in fermentation with recovery of substances released into the atmosphere, according to claim 2, which is **characterized by** the fact that the injections of CO₂ at low flow and at high flow are determined by the parameters of the density of the must contained in the fermentation tank; and at least one other parameter selected from between temperature and redox potential.

8. Method of maceration of wines in fermentation with recovery of substances released into the atmosphere, according to claim 1, which is **characterized by** the fact that the fermentation speed is modified by acting on the temperature of the fermentation tank and/or providing oxygen based on the measurements obtained by field sensors on parameters of the density of the must contained in the fermentation tank; and at least one other selected parameter of between, temperature and redox potential.

9. Method of maceration of wines in fermentation with recovery of substances released into the atmosphere, according to claim 3, which is **characterized by** the fact that the maximum fermentation temperature of red wines is 28ºC.

10. Method of maceration of wines in fermentation with recovery of substances released into the atmosphere, according to claim 1 or 2, which is **characterized by** the fact that, in the second stage of fermentation of white wines, both CO₂ and volatile substances are injected into the fermentation tank at a low flow rate.

11. Method of maceration of wines in fermentation with recovery of substances released into the atmosphere, according to claim 10, which is **characterized by** the fact that the maximum fermentation temperature of white wines is 20ºC.

12. Method of maceration of wines in fermentation with recovery of substances released into the atmosphere, according to any of the above claims that is **characterized by** the fact that the CO₂ excess (not injected into tanks in the pre-fermentation or fermentation phase) is reused in the winery itself for the inerting of equipment, the protection of the grapes in the refrigeration of pellets at the entrance of the winery or for the generation of eco-fuels.

13. A system for macerating wines in fermentation with recovery of substances released into the atmosphere, to carry out a method according to any of the above claims, which is **characterised by** the fact that it comprises:
- one or more fermentation tanks (1), where the must ferments and each of which is connected to suction media (2) where the CO₂ is aspirated together with the other volatile substances generated during fermentation;
- a cyclone filter (4) connected to the outlet of the suction media (2), to separate:
- The CO₂ in gaseous form is conveyed to a CO₂ tank (7) where it is stored compressed and,
- Condensed volatile substances that are conveyed to a condensates tank (8);
- means of injection (13) of separated CO₂ and condensed volatile substances, as well as any oxygen supply (14), comprising a series of automated valves for high and low flow rates with their pressure regulators, placed at the bottom of the tanks (1);
- a dosing pump (9) that pumps the condensed volatile substances from the condensates tank (8) to one or more fermentation tanks (1).
- a measuring station (10) equipped with density, temperature and redox potential sensors that is connected to the fermentation tank(s) (1) by means of an automatic sampler (11), which analyses the samples taken from the fermentation tank(s) (1) and where these data are measured and recorded; and
- a PLC or programmable controller (12) in connection with the measuring station (10) which is programmable, where the data recorded by the measuring station (10) arrives; wherein, this PLC or programmable controller (12), being in connection with the injection means (13), the dosing pump (9) and the external oxygen supply (14), regulates the injection of CO₂ flow, the injection of condensed volatile substances and the possible supply of oxygen, within the fermentation tank(s) (1), according to pre-established times according to a previous programming and based on parameters of density, temperature or redox potential.

14. A system for macerating wines during fermentation with recovery of substances released into the atmosphere, according to claim 13, which is **characterised by** the fact that it comprises a heat exchanger (3) located at the inlet of the cyclone filter (4).

15. A system for macerating wines in fermentation with recovery of substances released into the atmosphere, according to claim 13 or 14, which is **characterised by** the fact that it also includes:
- a buffer tank (5) for low-pressure CO₂ storage in connection with the suction media (2) and cyclone filter (4), and;
- a compressor (6) which extracts and compresses the CO₂ stored in the buffer tank (5) and conducts it to the CO₂ tank (7) where the compressed CO₂ is stored at higher pressure, until this carbon dioxide is injected back into the fermentation tank(s) (1) via the injection system (13).

16. A system for macerating wines during fermentation with recovery of substances released into the atmosphere, according to claim 13, which is **characterised by** the fact that the suction media (2) consist of:
- a suction turbine consisting of a side-channel vacuum pump in systems configured to move large volumes of CO₂; o
- an oil- and silicone-free, food-grade compressor in systems configured to move small volumes of CO₂.

17. A system for macerating wines during fermentation with recovery of substances released into the atmosphere, according to claims 13-14, which is **characterised by** the fact that the cyclone filter (4) recovers between 50-60% of the condensates lost in the fermentation of the must in the fermentation tank(s) (1), reaching up to 80% when preceded by an exchanger (3) to cool the fluid.

18. Wine maceration system in fermentation with recovery of substances released into the atmosphere, according to claim 13, which is **characterised by** the fact that the buffer tank (5) recovers between 20-30% of the condensates lost in the fermentation of the must in the fermentation tank(s) (1) due to the cooling coil inside.

19. System of maceration of wines in fermentation with recovery of substances released into the atmosphere according to claim 13 which is **characterized by** the fact that the CO₂ excess is liquefied for storage and subsequent use in different winery or external processes.
